# EUROPEAN PATENT SPECIFICATION

(11) **EP 4 225 403 B1**
(45) Date of publication and mention of the grant of the patent: **03.12.2025**
(21) Application number: 21783312.8
(22) Date of filing: 04.10.2021
(51) Int. Cl.: A61M 5/20, A61M 5/315, A61M 5/31, A61M 5/48

(54) **INJECTION DEVICE WITH SENSOR ASSEMBLY**
INJEKTIONSVORRICHTUNG MIT SENSORANORDNUNG
DISPOSITIF D'INJECTION DOTÉ D'UN ENSEMBLE CAPTEUR

(30) Priority: 05.10.2020 EP 20306153
(43) Date of publication of application: 16.08.2023
(73) Proprietor: Becton Dickinson France, 38800 Le Pont-de-Claix (FR)
(72) Inventor: LE GAL REDON, Patrick, 38170 SEYSSINET PARISET (FR)
(74) Representative: Germain Maureau
(86) International application number: PCT/EP2021/077268
(87) International publication number: WO 2022/073916

(56) References cited:
- EP-A1- 3 162 396
- WO-A1-2019/149868
- US-A1- 2012 089 114
- US-A1- 2016 259 913

## Description

### BACKGROUND

### 1. Field

The present disclosure relates generally to an injection device and, in some non-limiting embodiments or aspects, to a spring-based injection device including a sensor assembly.

### 2. Technical Considerations

A wide variety of injection devices for fluid injections are currently commercially available. US2016259913A1, WO2019149868A1, EP3162396A1 and US2012089114A1 describe examples of drug delivery devices. However, many of these devices do not provide any mechanism to capture and/or transfer injection related data.

### SUMMARY

The invention lies in the injection device of appended claim 1. Further embodiments are given in the dependent claims. Generally, this disclosure provides improved systems, devices, products, apparatus, and/or methods for capturing and/or transferring injection related data.

According to some non-limiting embodiments or aspects, provided is an injection device for delivering a dose of medicament to a user, the injection device including: a container having an open proximal end, a substantially closed distal end, and a reservoir defined between the open proximal end and the substantially closed distal end, the reservoir including the medicament; a mobile component provided in the container and movable with respect to the container, a movement of the mobile component causing the medicament to be expelled from the container; a spring connected to the mobile component, wherein the spring is configured to cause the mobile component to move within the container to cause the medicament to be expelled from the container; a sensor connected to an end of the spring, wherein the sensor is configured to measure a parameter associated with an injection operation of the injection device; and a wireless communication device configured to communicate, to a computing device, information associated with the injection operation of the injection device.

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of limits. As used in the specification and the claims, the singular form of "a," "an," and "the" include plural referents unless the context clearly dictates otherwise.

### BRIEF DESCRIPTION OF THE DRAWINGS

Additional advantages and details of embodiments or aspects of the present disclosure are explained in greater detail below with reference to the exemplary embodiments that are illustrated in the accompanying schematic figures, in which:
FIG. 1A is a diagram of non-limiting embodiments or aspects of an environment in which systems, devices, products, apparatus, and/or methods, described herein, may be implemented;
FIG. 1B is a perspective view of non-limiting embodiments or aspects of components of one or more devices and/or one or more systems of FIG. 1A;
FIG. 2 is a diagram of non-limiting embodiments or aspects of components of one or more devices and/or one or more systems of FIGS. 1A and 1B;
FIGS. 3A-3C are perspective views of implementations of non-limiting embodiments or aspects of components of one or more devices and/or one or more systems of FIGS. 1A, 1B, and 2;
FIG. 4 is a diagram of non-limiting embodiments or aspects of components of one or more devices and/or one or more systems of FIGS. 1A, 1B, 2, and 3A-3C.
FIG. 5A is a side view, a top view, and a section view taken along the line AA of non-limiting embodiments or aspects of an adapter plate; and
FIGS. 5B and 5C are perspective views of non-limiting embodiments or aspects of an adapter plate for improving an interface of a sensor with an end of a spring.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to the present disclosure as it is oriented in the drawing figures. However, it is to be understood that the present disclosure may assume various alternative variations and step sequences, except where expressly specified to the contrary. It is also to be understood that the specific devices and processes illustrated in the attached drawings, and described in the following specification, are simply exemplary embodiments or aspects of the present disclosure. Hence, specific dimensions and other physical characteristics related to the embodiments or aspects of the embodiments disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least in partially on" unless explicitly stated otherwise.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible.

It will be apparent that systems and/or methods, described herein, can be implemented in different forms of hardware, software, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

As used herein, the term "computing device" or "computer device" may refer to one or more electronic devices that are configured to directly or indirectly communicate with or over one or more networks. The computing device may be a mobile device, a desktop computer, or the like. Furthermore, the term "computer" may refer to any computing device that includes the necessary components to receive, process, and output data, and normally includes a display, a processor, a memory, an input device, and a network interface. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUls) with which a user may interact, either directly or indirectly (e.g., through a keyboard, mouse, touchscreen, etc.).

As used herein, the term "server" may refer to or include one or more processors or computers, storage devices, or similar computer arrangements that are operated by or facilitate communication and processing for multiple parties in a network environment, such as the Internet, although it will be appreciated that communication may be facilitated over one or more public or private network environments and that various other arrangements are possible. Further, multiple computers, e.g., servers, or other computerized devices, such as POS devices, directly or indirectly communicating in the network environment may constitute a "system," such as a merchant's POS system. As used herein, the term "data center" may include one or more servers, or other computing devices, and/or databases.

As used herein, the term "mobile device" may refer to one or more portable electronic devices configured to communicate with one or more networks. As an example, a mobile device may include a cellular phone (e.g., a smartphone or standard cellular phone), a portable computer (e.g., a tablet computer, a laptop computer, etc.), a wearable device (e.g., a watch, pair of glasses, lens, clothing, and/or the like), a personal digital assistant (PDA), and/or other like devices. The terms "client device" and "user device," as used herein, refer to any electronic device that is configured to communicate with one or more servers or remote devices and/or systems. A client device or user device may include a mobile device, a network-enabled appliance (e.g., a network-enabled television, refrigerator, thermostat, and/or the like), a computer, and/or any other device or system capable of communicating with a network.

As used herein, the term "application" or "application program interface" (API) refers to computer code, a set of rules, or other data sorted on a computer-readable medium that may be executed by a processor to facilitate interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, etc.).

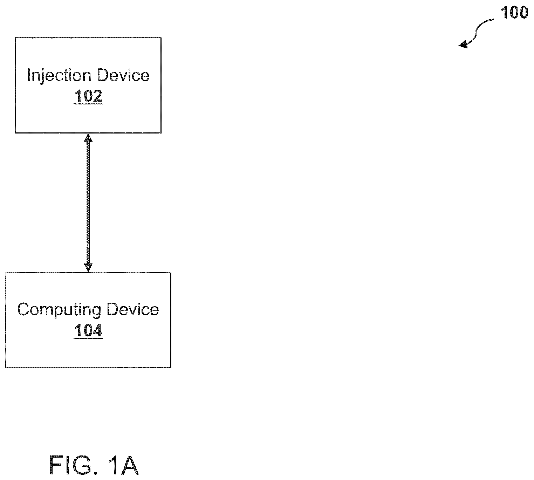

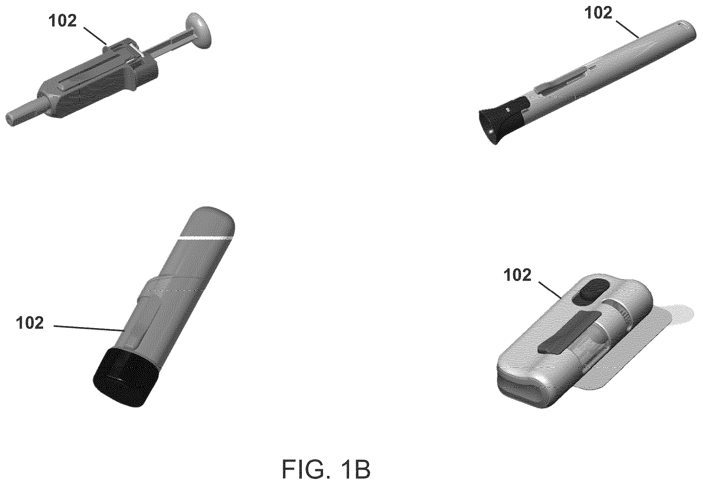

Referring to FIG. 1A, non-limiting embodiments or aspects of an environment 100 in which systems, devices, products, apparatus, and/or methods, as described herein, may be implemented is shown. As shown in FIG. 1, environment 100 may include injection device 102 and/or computing device 104.

Injection device 102 is configured to perform an injection operation to deliver a dose of medicament to a user. Injection device 102 may include injection devices with or without injection needles. Injection device 102 may be a disposable type injection device (e.g., a single use injection device, etc.) or a re-usable type injection device. Injection device 102 may include an auto injector, a pen injector, a wearable injector, a passive needle guard, and/or the like. For example, injection device 102 may include the BD Intevia^{™} autoinjector, the BD Libertas^{™} wearable autoinjector, the BD Sonic^{™} autoinjector, the BD UltraSafe Plus^{™} passive needle guard for a pre-filled ISO standard glass syringe, and/or the like. As an example, FIG. 1B shows various non-limiting embodiments or aspects of injection device 102.

Computing device 104 may include one or more devices capable of receiving information and/or data from injection device 102 (e.g., from sensor module 210, etc.) and/or communicating information and/or data to injection device 102 (e.g., to sensor module 210, etc.). For example, computing device 104 may include a computing device, a server, a group of servers, a mobile device, a group of mobile devices, a bridge, a router, a hub, and/or the like. In some non-limiting embodiments or aspects, computing device 104 includes one or more computing devices, chips, contactless transmitters, contactless transceivers, NFC transmitters, RFID transmitters, contact based transmitters, and/or the like that enables computing device 104 to receive information directly from and/or communicate information directly to injection device (e.g., wireless communication device 216, etc.) via a short range wireless communication connection (e.g., a communication connection that uses NFC protocol, a communication connection that uses Radio-frequency identification (RFID), a communication connection that uses a Bluetooth^{®} wireless technology standard, and/or the like). In some non-limiting embodiments or aspects, computing device 104 may include and/or upload information and/or data to an electronic data management system, such as a hospital record system, a system used during a clinical trial to collect the trial related information, and/or the like, and/or to another computing device.

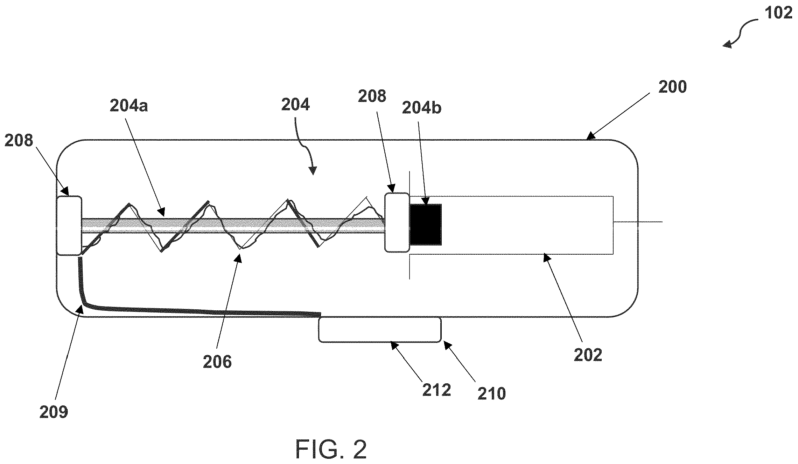

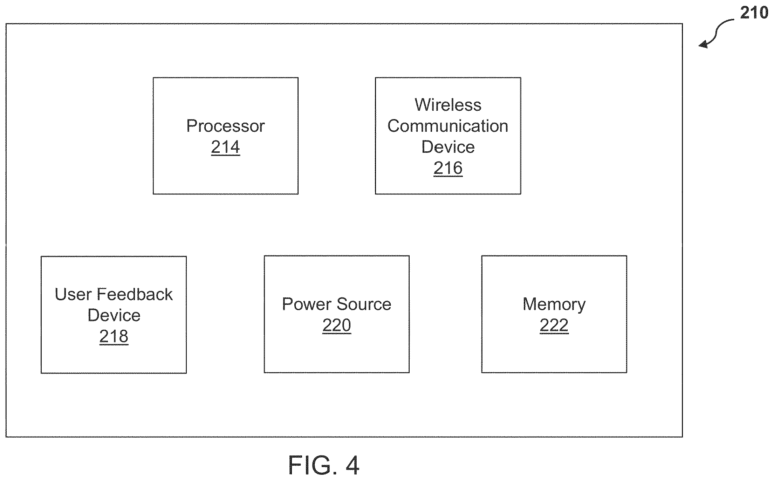

Referring now to FIGS. 2, 3A-3C, and 4, injection device 102 includes housing 200, container 202, mobile component 204 (e.g., plunger rod 204a, stopper 204b, etc.), spring 206 (e.g., a coil spring, etc.), sensor 208, electrically conductive trace 209, and/or sensor module 210. For example, container 202, mobile component 204 (e.g., plunger rod 204a, stopper 204b, etc.), spring 206, sensor 208, electrically conductive trace 209, and/or sensor module 210 may be housed within and/or connected to housing 200 of injection device 102. Sensor module 210 may include housing 212, processor 214, wireless communication device 216, user feedback device 218, power source 220, and/or memory 222. For example, processor 214, wireless communication device 216, user feedback device 218, power source 220, and/or memory 222 may be housed within housing 212 (e.g., on a printed circuit board (PCB) within housing 212, etc.). Electrically conductive trace 209 connects sensor module 210 (e.g., one or more components of sensor module 210, etc.) to sensor 208. For example, sensor 208, electrically conductive trace 209, and/or sensor module 210 may form a sensor assembly.

Container 202 has an open proximal end, a substantially closed distal end, and a reservoir defined between the open proximal end and the substantially closed distal end. The reservoir may include a medicament.

Mobile component 204 is provided in container 202 (e.g., received in the open proximal end of container 202, etc.) and movable with respect to container 202. A movement of movable component 204 causes the medicament to be expelled from the container in an injection operation (e.g., expelled from the substantially closed end of container 202 via a needle connected thereto, etc.). As an example, mobile component 204 may include plunger rod 204a and stopper 204b.

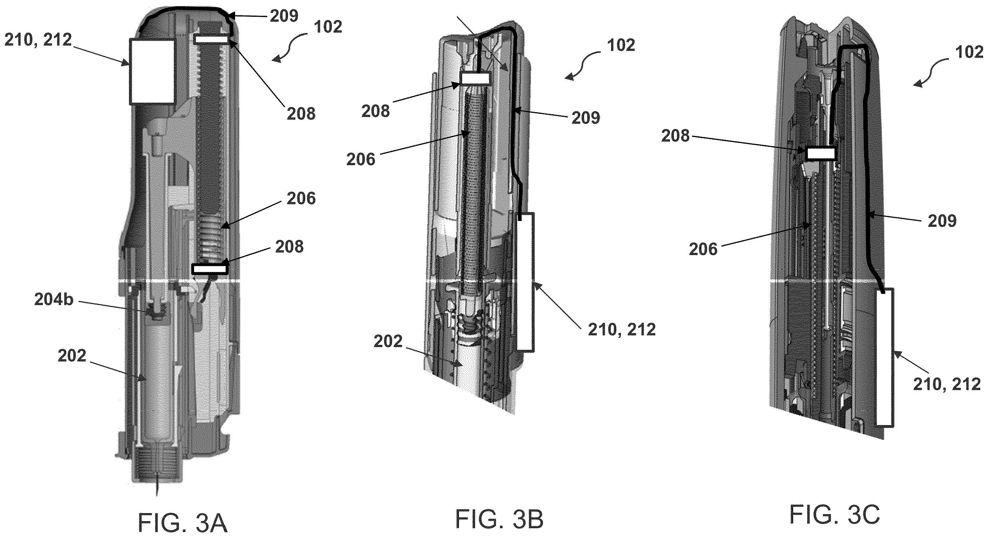

Spring 206 is connected to mobile component 204. Spring 206 is configured to cause mobile component 204 to move within container 202 to cause the medicament to be expelled from container 202. As an example, spring 206 may be held in a compressed position until a trigger that initiates or triggers the injection operation (e.g., a button, a trigger, etc.) is actuated or displaced release spring 206 from its compressed position to cause mobile component 204, which is connected to spring 206, to move distally within container 202 to cause the medicament to be expelled from container 202. In such an example, spring 206 may expand to cause plunger rod 204a and stopper 204b to move distally within container 202. Although FIG. 2 shows spring 206 directly connected to plunger rod 204a and/or stopper 204b, non-limiting embodiments or aspects are not limited thereto, and spring 206 may be indirectly connected to plunger rod 204a and/or stopper 204b (e.g., via one or more other mobile components, etc.), for example, as shown in FIG. 3A.

Sensor 208 is connected to (e.g., directly connected to, directly attached to, integrated with, etc.) an end of spring 206. For example, sensor 208 may be connected to a distal end and/or a proximal end of spring 206. As an example, sensor 208 may be connected to an end of spring 206 in contact with stopper 204a opposite a base of spring 206. In such an example, sensor 208 may be in contact with stopper 204b. As an example, sensor 208 may be connected to another end of spring 206 that is opposite an end of spring 206 that is in contact with stopper 204b. In some non-limiting embodiments or aspects, a first sensor 208 (e.g., a force sensor, an accelerometer, etc.) may be connected to a first end of spring 206 and a second sensor 208 (e.g., an accelerometer, a force sensor, etc.) may be connected to a second end of spring 206 opposite the first end of spring 206.

Sensor 208 is configured to measure a parameter associated with an injection operation of injection device 102. Sensor 208 may include a force sensor, an accelerometer, or any combination thereof. For example, sensor 208 may include a force sensor (e.g., a strain gauge, an extensometer sensor, a piezoelectric sensor, etc.) configured to measure a force associated with an injection operation of injection device 102. As an example, sensor 208 may measure a mechanical deformation of a force applied to an object where sensor 208 is positioned (e.g., a force applied at an end of spring 206, a force applied to mobile component 204, etc.) and transform the measured parameter into an electrical signal for processing and/or communication at sensor module 210. For example, sensor 208 may include an accelerometer configured to measure an acceleration associated with the injection operation of the injection device. As an example, sensor 208 may measure a speed of displacement of an object (e.g., an end of spring 206, mobile component 204, etc.) on which sensor 208 is positioned along one or more axes of the object and transform the measured parameter into an electrical signal for processing and/or communication at sensor module 210.

As shown in FIG. 3A, in some non-limiting embodiment or aspects, sensor module 210 (e.g., housing 212, etc.) is included and/or housed within (e.g., completely within, integrated with, etc.) housing 200. In such an example, sensor 208 may be spaced apart from sensor module 210 (e.g., housing 212, etc.) within housing 200. In some non-limiting embodiments or aspects, as shown in FIGS. 2, 3B and 3C, sensor module 210 (e.g., housing 212, etc.) is connected or attached to an external surface of housing 200. For example, sensor module 210 (e.g., housing 210, etc.) and/or injection device 102 (e.g., housing 200, etc.) may include a mechanical interface configured to attach (e.g., removably attach, permanently attach, etc.) housing 210 to an external surface of housing 200. As an example, the mechanical interface may include a clip, a snap fit connection, an adhesive, and/or other mechanical interface.

Electrically conductive trace 209 connects processor 214, wireless communication device 216, user feedback device 218, power source 220, and/or memory 222 to sensor 208. In some non-limiting embodiments or aspects, at least a portion of electrically conductive trace 209 is integrated in housing 200 (e.g., integrated in a plastic molded housing of injection device 102, etc.). According to the claimed invention, spring 206 (e.g., a metal spring, an electrically conductive spring, etc.) forms at least a portion of electrically conductive trace 209. As shown in FIG. 2, spring 206 acts as an electrically conductive trace that electrically connects sensor 208 adjacent stopper 204b to electrically conductive trace 209 (e.g., extending between a base of spring 206 and sensor module 210, etc.) and sensor module 210 (e.g., one or more components of sensor module 210, etc.). As an example, spring 206 may communicate a signal from sensor 208 to electrically conductive trace 209 and sensor module 210 (e.g., one or more components of sensor module 210, etc.).

Processor 214 may be programmed and/or configured to determine, based on a signal or sensor data (e.g., a measured parameter, a measured force, a measured acceleration, etc.) received from sensor 208, information associated with an injection operation of injection device 102. For example, processor 214 may be programmed and/or configured to receive acquisition signals from sensor 208 and, based on the received acquisition signals, processor 214 may be programmed and/or configured to perform signal processing (e.g., filtering, pattern detection etc.) on the received signals, perform threshold level(s) extraction, and/or determine information associated with an injection operation of injection device 102. As an example, processor 214 may be programmed and/or configured to compare a signal received from sensor 208 to one or more reference signals (e.g., stored in memory 222, etc.) to determine at least one of the following: a start time of the injection operation, a duration of the injection operation, a speed of the injection operation, a back pressure profile of the injection operation, or any combination thereof. In some non-limiting embodiments or aspects, processor 146 may include a low power microcontroller unit (MCU). In some non-limiting embodiments or aspects, information associated with an injection operation of injection device 102 includes at least one of the following: a measured parameter, a measured force, a measured acceleration, a start time of the injection operation, a duration of the injection operation, a speed of the injection operation, a back pressure profile of the injection operation, or any combination thereof. Processor 214 may store information associated an injection operation of injection device 102 in memory 222.

Wireless communication device 216 is configured to wirelessly communicate with computing device 104. Further, wireless communication device 216 is configured to communicate information associated with an injection operation of injection device 102 to computing device 104. In some non-limiting embodiments or aspects, wireless communication device 216 includes one or more computing devices, chips, contactless transmitters, contactless transceivers, NFC transmitters, RFID transmitters, contact based transmitters, Bluetooth transceivers^{®} and/or the like that enables wireless communication device 216 to receive information directly from and/or communicate information directly to computing device 104 via a short range wireless communication connection (e.g., a communication connection that uses NFC protocol, a communication connection that uses Radio-frequency identification (RFID), a communication connection that uses a Bluetooth^{®} wireless technology standard, and/or the like).

In some non-limiting embodiments or aspects, computing device 104 may be programmed and/or configured to determine, based on a signal or sensor data (e.g., a measured parameter, a measured force, a measured acceleration, etc.) received from sensor 208 via wireless communication device 216, information associated with an injection operation of injection device 102. For example, computing device 104 may be programmed and/or configured to receive acquisition signals from sensor 208 via wireless communication device 216 and, based on the received acquisition signals, computing device 104 may be programmed and/or configured to perform signal processing (e.g., filtering, pattern detection etc.) on the received signals, perform threshold level(s) extraction, and/or determine information associated with an injection operation of injection device 102. As an example, computing device 104 may be programmed and/or configured to compare a signal received from sensor 208 via wireless communication device 216 to one or more reference signals (e.g., stored in a memory, etc.) to determine at least one of the following: a start time of the injection operation, a duration of the injection operation, a speed of the injection operation, a back pressure profile of the injection operation, or any combination thereof.

User feedback device 218 may be configured to provide an indication and/or information associated with an injection operation of injection device 102 to a user. For example, user feedback device 150 may include at least one of the following: a display, a light-emitting diode (LED), an audio output device (e.g., a buzzer, a speaker, etc.), or any combination thereof. In some non-limiting embodiments or aspects, user feedback device 218 may include an user input device configured to receive user input from a user.

Power source 220 may be configured to power sensor 208, processor 214, wireless communication device 216, user feedback device 218, and/or memory 222. For example, power source 220 may include a battery (e.g., a rechargeable battery, a disposable battery, etc.) and/or an energy harvester (e.g., a thermoelectric energy harvester, a photovoltaic energy harvester, a piezoelectric energy harvester, etc.)

Memory 222 may be configured to store information associated with an injection operation of injection device 102 in memory 222. In some non-limiting embodiments or aspects, memory 222 may store calibration data associated with one or more calibration settings of sensor 208.

The number and arrangement of devices and systems shown in FIGS. 1A, 1B, 2, 3A-3C, and 4 is provided as an example. There may be additional devices and/or systems, fewer devices and/or systems, different devices and/or systems, or differently arranged devices and/or systems than those shown in FIGS. 1A, 1B, 2, 3A-3c, and 4. Furthermore, two or more devices and/or systems shown in FIGS. 1A, 1B, 2, 3A-3c, and 4 may be implemented within a single device and/or system, or a single device and/or system shown in FIGS. 1A, 1B, 2, 3A-3c, and 4 may be implemented as multiple, distributed devices and/or systems. Additionally, or alternatively, a set of devices and/or systems (e.g., one or more devices or systems) of environment 100 may perform one or more functions described as being performed by another set of devices and/or systems of environment 100.

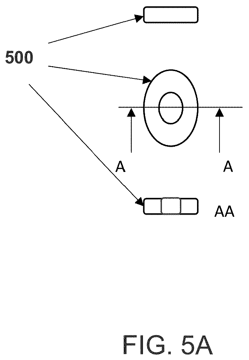

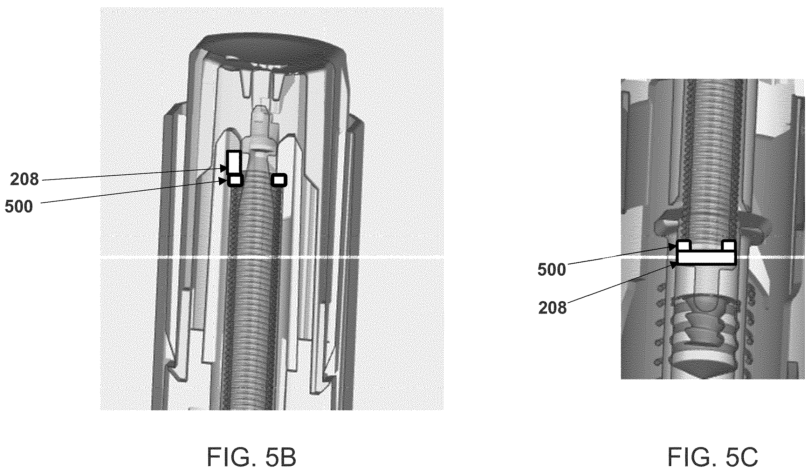

Referring now to FIG. 5A, FIG. 5A is a side view, a top view, and a section view taken along the line AA of non-limiting embodiments or aspects of adapter plate 500 for sensor 208. As shown in FIG. 5A, adapter plate 500 may have a shape corresponding to an end of spring 206. For example, adapter plate 500 may have a hollow cylinder shape. As an example, and referring now to FIGS. 5B and 5C, FIGS. 5B and 5C show perspective views of adapter plate 500 including a hollow cylinder shape to improve an interface of sensor 208 with an end of spring 206, which may enable more accurate parameter measurement by sensor 208. In some non-limiting embodiments or aspects, adapter plate 500 may be a separate component from sensor 208 (e.g., sensor 208 may be connected to adapter 500, etc.) at an end of spring 206. In some non-limiting embodiments or aspects, sensor 208 and may be fully integrated within adapter plate 500 as single component having a shape corresponding to an end of spring 206 of injection device 102.

Although embodiments or aspects have been described in detail for the purpose of illustration and description, it is to be understood that such detail is solely for that purpose and that embodiments or aspects are not limited to the disclosed embodiments or aspects, but, on the contrary, are intended to cover modifications and equivalent arrangements that are within the scope of the appended claims. For example, it is to be understood that the present disclosure contemplates that, to the extent possible, one or more features of any embodiment or aspect can be combined with one or more features of any other embodiment or aspect.

## Claims

1. An injection device (102) for delivering a dose of medicament to a user, the injection device comprising:
a container (202) having an open proximal end, a substantially closed distal end, and a reservoir defined between the open proximal end and the substantially closed distal end, the reservoir including the medicament;
a mobile component (204) provided in the container (202) and movable with respect to the container (202), a movement of the mobile component (204) causing the medicament to be expelled from the container (202);
a spring (206) connected to the mobile component (204), wherein the spring (206) is configured to cause the mobile component (204) to move within the container (202) to cause the medicament to be expelled from the container (202);
a sensor (208) connected to an end of the spring (206), wherein the sensor (208) is configured to measure a parameter associated with an injection operation of the injection device (102); and
a wireless communication device (216) configured to communicate, to a computing device (104), information associated with the injection operation of the injection device (102); and
an electrically conductive trace (209) that connects the sensor (208) to the wireless communication device (216);
**characterized in that**
the spring (206) forms at least a portion of the electrically conductive trace (209) that connects the sensor (208) to the wireless communication device (216).

2. The injection device of claim 1, wherein the mobile component (204) includes a plunger rod (204a) and a stopper (204b).

3. The injection device of claim 2, wherein the sensor (208) connected to the end of the spring (206) is in contact with the stopper (204b).

4. The injection device of claim 2, wherein the end of the spring (206) connected to the sensor (208) is opposite another end of the spring (206) in contact with the stopper (204b).

5. The injection device of claim 1, further comprising:
a first housing (200) including the container (202), the mobile component (204), the spring (206), the sensor (208), and the wireless communication device (216), wherein the sensor (208) is spaced apart from the wireless communication device (216) within the housing (200).

6. The injection device of claim 1, further comprising:
a first housing (200) including the container (202), the mobile component (204), the spring (206), and the sensor (208); and
a second housing (212) including the wireless communication device (216), wherein the second housing (212) is connected to an external surface of the first housing (200).

7. The injection device of claim 1, further comprising:
a battery (220) configured to provide power to the sensor (208) via the electrically conductive trace (209).

8. The injection device of claim 1, wherein the sensor (208) includes an adapter plate (500) having a hollow cylinder shape.

9. The injection device of claim 1, further comprising:
one or more processors (214) programmed and/or configured to determine, based on the measured parameter, the information associated with the injection operation of the injection device (102).

10. The injection device of claim 9, wherein the information associated with the injection operation of the injection device (102) includes at least one of the following: a start time of the injection operation, a duration of the injection operation, a speed of the injection operation, a back pressure profile of the injection operation, or any combination thereof.

11. The injection device of claim 1, further comprising:
a user feedback device (218), wherein the user feedback device (218) includes at least one of the following: a display, a light-emitting diode (LED), an audio output device, or any combination thereof.

12. The injection device of claim 1, wherein the sensor includes a force sensor configured to measure a force associated with the injection operation of the injection device (102).

13. The injection device of claim 1, wherein the sensor includes an accelerometer configured to measure an acceleration associated with the injection operation of the injection device (102).

## Patentansprüche

1. Einspritzvorrichtung (102) zum Verabreichen einer Medikamentendosis an einen Benutzer, wobei die Einspritzvorrichtung Folgendes umfasst:
einen Behälter (202), der ein offenes proximales Ende, ein weitgehend geschlossenes distales Ende und ein zwischen dem offenen proximalen Ende und dem weitgehend geschlossenen distalen Ende definiertes Reservoir aufweist, wobei das Reservoir das Medikament enthält;
eine in dem Behälter (202) vorgesehene bewegliche Komponente (204), die sich relativ zu dem Behälter (202) bewegen lässt, wobei eine Bewegung der beweglichen Komponente (204) dazu führt, dass das Medikament aus dem Behälter (202) ausgestoßen wird;
eine Feder (206), die mit der beweglichen Komponente (204) verbunden ist, wobei die Feder (206) so eingerichtet ist, dass sie die bewegliche Komponente (204) dazu veranlasst, sich innerhalb des Behälters (202) zu bewegen, um das Medikament aus dem Behälter (202) auszustoßen;
einen Sensor (208), der mit einem Ende der Feder (206) verbunden ist, wobei der Sensor (208) so eingerichtet ist, dass er einen Parameter misst, der mit einem Einspritzvorgang der Einspritzvorrichtung (102) in Verbindung steht; und
eine drahtlose Kommunikationsvorrichtung (216), die so eingerichtet ist, dass sie Informationen in dem Zusammenhang mit dem Einspritzvorgang der Einspritzvorrichtung (102) an eine Rechenvorrichtung (104) übermittelt; und
eine elektrisch leitfähige Spur (209), die den Sensor (208) mit der drahtlosen Kommunikationsvorrichtung (216) verbindet;
**dadurch gekennzeichnet, dass**
die Feder (206) mindestens einen Teil der elektrisch leitfähigen Spur (209) bildet, die den Sensor (208) mit der drahtlosen Kommunikationsvorrichtung (216) verbindet.

2. Einspritzvorrichtung nach Anspruch 1, wobei die bewegliche Komponente (204) eine Kolbenstange (204a) und einen Anschlag (204b) enthält.

3. Einspritzvorrichtung nach Anspruch 2, wobei der mit dem Ende der Feder (206) verbundene Sensor (208) mit dem Anschlag (204b) in Kontakt steht.

4. Einspritzvorrichtung nach Anspruch 2, wobei das Ende der Feder (206), das mit dem Sensor (208) verbunden ist, dem anderen Ende der Feder (206) gegenüberliegt, das mit dem Anschlag (204b) in Kontakt steht.

5. Einspritzvorrichtung nach Anspruch 1, ferner umfassend:
ein erstes Gehäuse (200), das den Behälter (202), die bewegliche Komponente (204), die Feder (206), den Sensor (208) und die drahtlose Kommunikationsvorrichtung (216) enthält, wobei der Sensor (208) von der drahtlosen Kommunikationsvorrichtung (216) innerhalb des Gehäuses (200) beabstandet ist.

6. Einspritzvorrichtung nach Anspruch 1, ferner umfassend:
ein erstes Gehäuse (200), das den Behälter (202), die bewegliche Komponente (204), die Feder (206) und den Sensor (208) enthält; und
ein zweites Gehäuse (212), das die drahtlose Kommunikationsvorrichtung (216) enthält, wobei das zweite Gehäuse (212) mit einer Außenfläche des ersten Gehäuses (200) verbunden ist.

7. Einspritzvorrichtung nach Anspruch 1, ferner umfassend:
eine Batterie (220), die so eingerichtet ist, dass sie den Sensor (208) über die elektrisch leitfähige Spur (209) mit Strom versorgt.

8. Einspritzvorrichtung nach Anspruch 1, wobei der Sensor (208) eine Adapterplatte (500) enthält, der die Form eines Hohlzylinders aufweist.

9. Einspritzvorrichtung nach Anspruch 1, ferner umfassend:
einen oder mehrere Prozessoren (214), die programmiert und/oder eingerichtet sind, um, basierend auf dem gemessenen Parameter, die Informationen zu bestimmen, die mit dem Einspritzvorgang der Einspritzvorrichtung (102) verbunden sind.

10. Einspritzvorrichtung nach Anspruch 9, wobei die mit dem Einspritzvorgang der Einspritzvorrichtung (102) verbundenen Informationen mindestens eine der folgenden enthalten:
eine Startzeit des Einspritzvorgangs, eine Dauer des Einspritzvorgangs, eine Geschwindigkeit des Einspritzvorgangs, ein Gegendruckprofil des Einspritzvorgangs oder eine beliebige Kombination davon.

11. Einspritzvorrichtung nach Anspruch 1, ferner umfassend:
eine Benutzerrückmeldevorrichtung (218), wobei die Benutzerrückmeldevorrichtung (218) mindestens eines der folgenden Elemente enthält: eine Anzeige, eine Leuchtdiode (LED), eine Audioausgabevorrichtung oder eine beliebige Kombination davon.

12. Einspritzvorrichtung nach Anspruch 1, wobei der Sensor einen Kraftsensor enthält, der so eingerichtet ist, dass er die mit dem Einspritzvorgang der Einspritzvorrichtung (102) verbundene Kraft misst.

13. Einspritzvorrichtung nach Anspruch 1, wobei der Sensor einen Beschleunigungsmesser enthält, der so eingerichtet ist, dass er eine mit dem Einspritzvorgang der Einspritzvorrichtung (102) verbundene Beschleunigung misst.

## Revendications

1. Dispositif d'injection (102) pour administrer une dose de médicament à un utilisateur, le dispositif d'injection comprenant :
un récipient (202) ayant une extrémité proximale ouverte, une extrémité distale sensiblement fermée et un réservoir défini entre l'extrémité proximale ouverte et l'extrémité distale sensiblement fermée, le réservoir comprenant le médicament ;
un composant mobile (204) prévu dans le récipient (202) et mobile par rapport au récipient (202), un mouvement du composant mobile (204) entraînant l'expulsion du médicament du récipient (202) ;
un ressort (206) relié au composant mobile (204), où le ressort (206) est configuré pour amener le composant mobile (204) à se déplacer dans le récipient (202) afin d'entraîner l'expulsion du médicament du récipient (202) ;
un capteur (208) relié à une extrémité du ressort (206), où le capteur (208) est configuré pour mesurer un paramètre associé à une opération d'injection du dispositif d'injection (102) ; et
un dispositif de communication sans fil (216) configuré pour communiquer, à un dispositif informatique (104), des informations associées à l'opération d'injection du dispositif d'injection (102) ; et
une piste électroconductrice (209) qui relie le capteur (208) au dispositif de communication sans fil (216) ;
**caractérisé en ce que**
le ressort (206) forme au moins une partie de la piste électroconductrice (209) qui relie le capteur (208) au dispositif de communication sans fil (216).

2. Dispositif d'injection selon la revendication 1, dans lequel le composant mobile (204) comprend une tige de piston (204a) et un bouchon (204b).

3. Dispositif d'injection selon la revendication 2, dans lequel le capteur (208) relié à l'extrémité du ressort (206) est en contact avec le bouchon (204b).

4. Dispositif d'injection selon la revendication 2, dans lequel l'extrémité du ressort (206) reliée au capteur (208) est opposée à une autre extrémité du ressort (206) en contact avec le bouchon (204b).

5. Dispositif d'injection selon la revendication 1, comprenant en outre :
un premier boîtier (200) comprenant le récipient (202), le composant mobile (204), le ressort (206), le capteur (208) et le dispositif de communication sans fil (216), où le capteur (208) est espacé du dispositif de communication sans fil (216) dans le boîtier (200).

6. Dispositif d'injection selon la revendication 1, comprenant en outre :
un premier boîtier (200) comprenant le récipient (202), le composant mobile (204), le ressort (206) et le capteur (208) ; et
un second boîtier (212) comprenant le dispositif de communication sans fil (216), où le second boîtier (212) est relié à une surface externe du premier boîtier (200).

7. Dispositif d'injection selon la revendication 1, comprenant en outre :
une batterie (220) configurée pour fournir de l'énergie au capteur (208) via la piste électroconductrice (209).

8. Dispositif d'injection selon la revendication 1, dans lequel le capteur (208) comprend une plaque d'adaptation (500) ayant une forme de cylindre creux.

9. Dispositif d'injection selon la revendication 1, comprenant en outre :
un ou plusieurs processeurs (214) programmés et/ou configurés pour déterminer, sur la base du paramètre mesuré, les informations associées à l'opération d'injection du dispositif d'injection (102).

10. Dispositif d'injection selon la revendication 9, dans lequel les informations associées à l'opération d'injection du dispositif d'injection (102) comprennent au moins l'un de ce qui suit : l'heure de début de l'opération d'injection, une durée de l'opération d'injection, une vitesse de l'opération d'injection, un profil de contre-pression de l'opération d'injection, ou toute combinaison de ceux-ci.

11. Dispositif d'injection selon la revendication 1, comprenant en outre :
un dispositif de retour à l'utilisateur (218), où le dispositif de retour à l'utilisateur (218) comprend au moins l'un de ce qui suit : un dispositif d'affichage, une diode électroluminescente (LED), un dispositif de sortie audio, ou toute combinaison de ceux-ci.

12. Dispositif d'injection selon la revendication 1, dans lequel le capteur comprend un capteur de force configuré pour mesurer une force associée à l'opération d'injection du dispositif d'injection (102).

13. Dispositif d'injection selon la revendication 1, dans lequel le capteur comprend un accéléromètre configuré pour mesurer une accélération associée à l'opération d'injection du dispositif d'injection (102).
